Europäisches Patentamt

**European Patent Office**

Office européen des brevets

(19)

(11) Veröffentlichungsnummer: **0 274 094**
**B1**

(12) **EUROPÄISCHE PATENTSCHRIFT**

(45) Veröffentlichungstag der Patentschrift:
29.08.90

(51) Int. Cl.⁵: **A61F 2/36**

(21) Anmeldenummer: **87118668.0**

(22) Anmeldetag: **16.12.87**

(54) Zementfreie Endoprothese.

(30) Priorität: **03.01.87 DE 3700102**
**31.07.87 DE 3725387**

(43) Veröffentlichungstag der Anmeldung:
**13.07.88 Patentblatt 88/28**

(45) Bekanntmachung des Hinweises auf die Patenterteilung:
**29.08.90 Patentblatt 90/35**

(84) Benannte Vertragsstaaten:
**AT BE CH DE ES FR GB GR IT LI LU NL SE**

(56) Entgegenhaltungen:
**DE-B- 2 247 560**
**FR-A- 2 575 383**
**US-A- 3 024 785**
**US-A- 3 848 276**

(73) Patentinhaber: **Kallabis, Manfred, Dr., Falkenweg 24,
D-8960 Kempten(DE)**
Patentinhaber: **Gombert, Guido, Dr., Schillerstrasse 18,
D-8960 Kempten(DE)**

(72) Erfinder: **Kallabis, Manfred, Dr., Falkenweg 24,
D-8960 Kempten(DE)**
Erfinder: **Gomberg, Guido, Dr., Schillerstrasse 18,
D-8960 Kempten(DE)**

(74) Vertreter: **Hübner, Hans-Jürgen, Dipl.-Ing.,
Mozartstrasse 31, D-8960 Kempten/Allgäu(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine zementfreie Endoprothese besonders für Hüftgelenke, mit einer in den natürlichen Knochenkanal einzuschraubenden Schraubspindel, deren proximales Ende mit einem schräg zur Spindelachse vorgesehenen Hals für einen Gelenkkopf mechanisch verbunden ist.

Eine Endoprothese dieser Art ist aus der FR-A-2 575 383 bekannt. Der Kern der Gewindespindel muß die gesamte Last übertragen und hat daher einen möglichst großen Durchmesser. Da der Knochenkanal nicht geradlinig verläuft, sondern immer antekurviert und häufig variisiert ist, muß er aufgebohrt werden. Das bedeutet zonenweise Schwächungen des Knochens. Die Schraubspindel findet zwangsläufig ihre größte Verankerung in den geschwächten Knochenpartien. Infolge der Krümmung des Knochenkanals tritt der Gewindesteg der Schraubspindel längs breitflächiger Zonen mit dem Knochen überhaupt nicht in Eingriff. Eine gleichmäßige Krafteinleitung durch die Schraubspindel in den Femur ist nicht möglich. Da sich der proximale Femuranteil kelchförmig öffnet, steht die Schraubspindel in diesem Bereich leer bzw. faßt mit ihrem Gewindesteg allenfalls Spongiosa. Am distalen Ende schneidet der Gewindesteg in die Corticalis ein, die erheblich härter als die Spongiosa ist, weswegen diese Endoprothese im wesentlichen im distalen Bereich trägt. Daraus resultiert eine permanente proximale Entlastung mit Schwächung der dortigen Lastaufnahmestrukturen und es entsteht letztlich eine von proximal nach distal zunehmende Atrophie der Knochenstrukturen als biomechanische Antwort. Die Prothese lockert sich allmählich und versagt schließlich. Außerdem tritt eine Verstarrung des Oberschenkelknochens ein und sein natürliches Schwingungsverhalten geht verloren. Aufgrund dieser Nachteile und weil jede Rotationsstabilität fehlt, denn die bei der Schrittführung auftretenden Druck- und Rotationskräfte können die Schraubspindel unkontrolliert verdrehen, hat man diese bekannte Endoprothese, die übrigens von Mc Bride schon 1948 publiziert wurde, schon seit langer Zeit verworfen.

Die heute allgemein verwendeten Endoprothesen für Hüftgelenke verwenden einen axial in den Knochenkanal einschiebbaren unrunden Schaft, der querschnittsmäßig und mit gewisser Biegung in seiner Längsrichtung den entsprechenden Dimensionen eines Knochenkanals etwa angepaßt ist (DE-Z-"Mechanik und Prothesentypen, Münch.med. Wschr. 118 (1976) Nr. 22, Seite 697, Abb. 5). Diese Endoprothesen arbeiten nach dem Verkeilungsprinzip. Im proximalen Bereich sind zusätzliche Rippen vorgesehen, die entweder in Schaftlängsrichtung verlaufen oder als querlaufende Tragrippen ausgebildet sind. Letztere haben gegenüber ersteren noch den Nachteil, daß beim Einschlagen des Schaftes Spongiosa abgeschert wird. Diese Schaftprothesen sind zwar rotationsstabil, tragen jedoch ebenfalls lediglich im distalen Bereich in der Corticalis mit den vorstehend beschriebenen Nachteilen.

Aufgabe der Erfindung ist es, eine neuartige Endoprothese zu schaffen, die eine bessere Verankerung im Schenkelknochen und zwar hauptsächlich im proximalen Bereich gestattet.

Diese Aufgabe wird bei einer zementfreien Endoprothese der eingangs genannten Art durch die Merkmale des Kennzeichnungsteils des Patentanspruches 1 gelöst.

Der Schaft, dessen Konturen prinzipiell denen des bekannten Schaftes entsprechen braucht nicht mehr eingeschlagen sondern lediglich satt eingesetzt zu werden. Der Schaft hat hauptsächlich eine Führungsfunktion für die Schraubspindel. Im distalen Bereich unterstützt er lediglich die Lasteinleitung. Ggf. kann das distale Ende des Schaftes sogar bis auf die Hälfte der Länge bisher üblicher Schäfte gekürzt werden. Dank des sich in die Spongiosa und auch in die Corticalis einschneidenden breitflächigen Gewindesteges der Schraubspindel wird im proximalen Bereich der Prothese ein Formschluß erzielt. Der Schaft wirkt als Tragrahmen für die Schraubspindel, die dadurch mit einem geringen Kerndurchmesser ausgestattet werden kann, der wiederum für die breiflächige Ausbildung des Gewindesteges ursächlich ist. Jeglicher Substanzverlust des Knochens wird vermieden, da der Knochenkanal nicht mehr aufgebohrt zu werden braucht.

Anspruch 2 führt zu dem Vorteil, daß Spongiosa in die Hohlräume im Bereich der Fensteröffnung eintreten kann.

Mit dem Merkmal des Anspruchs 3 wird erreicht, daß der Gewindesteg mindestens in dem zum Hals führenden Schaftschenkel zusätzlich abgestützt wird.

Die Merkmale der Ansprüche 4 und 5 führen zu einer maximal großen Fensteröffnung bei ausreichender Stabilität der verbleibenden Schaftschenkel.

Anspruch 6 erlaubt eine einstückige Bauweise des Schaftes, wobei die integrierte Brücke mehrere Funktionen erfüllt. Einmal erhöht sie die Belastbarkeit des Schaftes und zum anderen dient sie der Führung der Spindel beim Einschrauben. Tatsächlich wird die Spindel ausschließlich durch die Brücke auf der genau vorgegebenen schraubenförmigen Bahn bewegt, sodaß das Einschrauben beinahe blind erfolgen kann. Der vorzugsweise selbstschneidend ausgebildete Gewindesteg schneidet sich zunehmend tiefer in die Spongiosa und schließlich in die Corticalis ein, bis das untere Ende des Kerns der Schraubspindel in ein Sackloch des Schaftes formschlüssig eingreift. Beide Enden der Schraubspindel sind damit am Schaft sicher gehaltert. Wenn es auch schon überraschend ist, daß eine mit einem schraubenförmigen Schlitz von mindestens 180°, vorzugsweise aber 360° Umfangswinkel versehene Brücke eine mechanische Verbindung zwischen den beiden Schaftschenkeln herstellt, die allen Druck-, Zug- und Biegekräften standhält, so ist noch bemerkenswerter, daß dies sogar bei der Ausführung gemäß Patentanspruch 7 erreicht wird, denn es verbleiben immer Materialstränge oberhalb und unterhalb des schraubenförmigen Schlitzes, die die beiden Schaftschenkel miteinander verbinden. Die Brücke braucht daher gegenüber dem sich nach distal anschließenden Schaftteil nur geringfügig wulstartig verbreitert zu werden.

Das Merkmal vom Patentanspruch 10 dient dazu, die Spindel in der Einbaustellung drehsicher festzuhalten. Das Loch für das Drehsicherungselement wird vorzugsweise so eingebracht, daß das Drehsicherungselement in eine Schneidausnehmung am äußeren Umfang des Gewindesteges der Schraubspindel eingreift.

Anhand der Zeichnung, die ein Ausführungsbeispiel darstellt, sei die Erfindung näher beschrieben.

Es zeigt

FIG. 1 eine Seitenansicht der Prothese,
FIG. 2 eine Frontalansicht der Prothese in Richtung des Halses und
FIG. 3 eine Querschnittsansicht der Prothese längs der Linie 3-3 der FIG. 2.

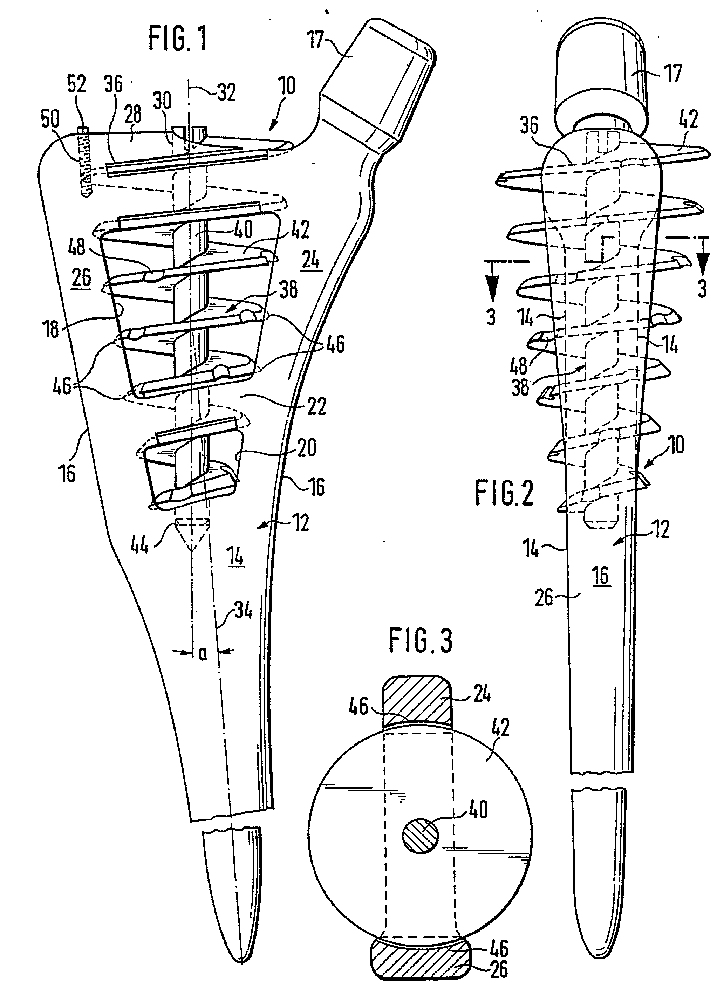

Eine Prothese 10 hat einen Schaft 12, dessen beide Breitseiten 14 sich vom proximalen Ende zum distalen Ende hin allmählich verjüngen. Die zwischen den beiden Schmalseiten 16 gemessene Dicke des Schaftes 12 verringert sich zum distalen Ende nur ganz geringfügig. Oben rechts (FIG.1) schließt sich an den Schaft 12 einstückig ein leicht konischer Hals 17 für einen Gelenkkopf an.

Im proximalen Bereich des Schaftes 12 befinden sich zwei Fensteröffnungen 18,20, die sich rechtwinklig zu den Breitseiten 14 vollständig durch den Schaft 12 hindurch erstrecken. Der zwischen den beiden Fensteröffnungen 18, 20 verbleibende Steg 22 dient der Stabilitätserhöhung. Zwischen den Schmalseiten 16 und den Fensteröffnungen 18, 20 werden zwei nach proximal divergierende Schenkel 24, 26 gebildet, die oben von einer Brücke 28 miteinander einstückig verbunden sind. Die Brücke 28 ist wulstartig um ein geringes Maß gegenüber den Breitseiten 14 des Schaftes 12 verdickt. Der vom Hals 17 abgewandte Schenkel 26 ist beidseitig breiter als der übrige Teil des Schaftes 12, womit eine Anpassung an die Querschnittsform des Knochenkanals erzielt wird.

In der Brücke 28 befindet sich eine Bohrung 30, deren Achse 32 mit der Schaftachse 34 einen Winkel a von etwa 5° bildet, derart, daß die Achse 32 gegenüber der Schaftachse 34 zum Hals 17 hin etwas geneigt ist. An die Bohrung 30 schließt sich nach außen ein schraubenförmiger Schlitz 36 an, der an den Deck- und Bodenseiten der Brücke 28 mündet, in die Verlängerungsbereiche der beiden Schenkel 24, 26 hineinragt und an den Breitseiten 14 des Schaftes 12 austritt. Trotz dieser Schlitzführung verbleiben Materialstränge in der Brücke 28, die die beiden Schenkel 24, 26 verbinden und zwar verlaufen diese Materialstränge in der einen Axialhälfte des Schaftes 12 oberhalb des Schlitzes und in der anderen Hälfte unterhalb des schraubenförmigen Schlitzes 36.

In den Schaft 12 ist eine Schraubspindel 38 längs der Achse 32 eingeschraubt, die aus einem kreiszylindrischen Kern 40 und einem breitflächigen sich von proximal nach distal konisch verjüngenden Gewindesteg 42 besteht. Der Konizitätswinkel beträgt 20°. Der Gewindesteg 42 hat eine fast konstante Dicke und eine konstante Steigung von 11° und es versteht sich, daß der auf schraubenförmigem Bahn liegende Schlitz 36 in der Brücke 28 mit diesen Dimensionen übereinstimmt. Der größte Durchmesser des Gewindesteges 42 beträgt 32mm und der kleinste Durchmesser am unteren Ende ist 13mm groß. Die axiale Länge des Schraubgewindes 42 ist etwa 52mm. Genaugenommen, ist die Dicke des Gewindesteges 42 nicht konstant, sondern die beiden Flanken des Gewindesteges bilden einen Winkel von etwa 5° miteinander, sodaß sich der Gewindesteg zur Wurzel hin geringfügig verdickt.

Die Schraubspindel 38 wird durch die Brücke 28 hindurch in den Schaft 12 eingeschraubt, wobei die Bohrung 30 und der schraubenförmige Schlitz 36 die Schraubspindel 38 von Anfang an auf einer schraubenförmigen Bahn zwangsweise führen. Nach einer Volldrehung der Schraubspindel 38 tritt der untere Abschnitt des Gewindesteges 42 bodenseitig aus der Brücke 28 aus und beim weiteren Einschrauben durchsetzt die Schraubspindel 38 allmählich zuerst die obere Fensteröffnung 18 und dann die unteres Fensteröffnung 20, bis das untere Ende des Kerns 40 der Schraubspindel 38 in ein zur Bohrung 30 koaxial liegendes Sackloch 44 eintritt. Gleichzeitig tritt der Außenrand des Gewindesteges 42 in schraubenförmige Nuten 46 ein, die an den Innenflächen der Schenkel 24, 26 eingearbeitet sind. Es versteht sich, daß sich an die unterste Nut 46 der Fensteröffnung 18 im Bereich des Versteifungssteges 22 ein schraubenförmiger Schlitz entsprechend dem Schlitz 36 anschließt, der zur oberen schraubenförmigen Nut im Schenkel 26 der Fensteröffnung 20 führt.

Im voll eingeschraubten Zustand der Schraubspindel 38 ist der Kern 40 beidendig in der Bohrung 30 und dem Sackloch 44 verankert und der obere Abschnitt des Gewindesteges 42 füllt den schraubenförmigen Schlitz 36 in der Brücke 28 aus und verläuft teilweise parallel zur Brückendeckfläche. Der Außenrand des Gewindesteges 42 greift im Bereich der Fensteröffnungen 18,20 in die schraubenförmigen Nuten 46, wodurch der breitflächige Gewindesteg 42 zusätzlich stabilisiert ist. Der Gewindesteg 42 weist am Außenrand eine Anzahl Hinterschneidungen 48 auf, die im Umfangsabstand von 90° angeordnet sind. An diesen Hinterschneidungen werden Schneidzähne gebildet, die den Gewindesteg 42 selbstschneidend machen.

In der Brücke 28 befindet sich ein Loch 50, in das ein Drehsicherungselement 52 in Form einer Sperrschraube eingeschraubt ist. Das Loch 50 ist genau so angeordnet, daß die Sperrschraube eine Hinterschneidung 48 des Gewindesteges 42 durchsetzt.

Die Fensteröffnungen 18, 20 sind prinzipiell trapezförmig ausgebildet, wobei der Trapezwinkel dem Konizitätswinkel des Gewindesteges 42 entspricht. Die oberen und unteren Begrenzungsflächen der Fensteröffnungen 18, 20 verlaufen in jeder der beiden Axialhälften des Schaftes 12 etwa parallel zum Gewindesteg 42. Die Nuten 46 in den Schenkeln 24, 26 haben somit jeweils dieselbe Nuttiefe.

## Patentansprüche

1. Zementfreie Endoprothese besonders für Hüftgelenke, mit einer in den natürlichen Knochenkanal einzuschraubenden, ein Außengewinde aufweisenden Schraubspindel (38) deren proximales Ende mit einem schräg zur Spindelachse (32) vorgesehenen Hals (17) für einen Gelenkkopf mechanisch verbunden ist, dadurch gekennzeichnet, daß die Schraubspindel (38) in einem, dem Knochenkanal querschnittsmäßig etwa angepaßten und sich zum distalen Ende hin verjüngenden Schaft (12) etwa in dessen Längsrichtung eingeschraubt ist, der zwei Breitseiten (14) und zwei diese verbindende Schmalseiten (16) aufweist, daß das Außengewinde als breitflächiger Gewindesteg (42) ausgebildet ist, der an beiden Breitseiten des Schaftes (12) aus diesem herausragt, und daß die Verbindung der Schraubspindel (38) mit dem Hals (17) durch den Schaft (12) erfolgt.

2. Endoprothese nach Anspruch 1, dadurch gekennzeichnet, daß der Schaft (12) mindestens eine, ihn durchsetzende Fensteröffnung (18; 20) aufweist, durch die sich die Schraubspindel (38) von proximal nach distal hindurch erstreckt, und daß zwischen dem Kern (40) der Schraubspindel (38) und den zwischen den Schmalseiten (16) des Schaftes (12) und der Fensteröffnung (18; 20) gebildeten Schenkeln (24,26) durchgängige Hohlräume verbleiben.

3. Endoprothese nach Anspruch 1 oder 2, dadurch gekennzeichnet, daß mindestens einer der zwischen den Schmalseiten (16) des Schaftes (12) und der Fensteröffnung (18; 20) gebildeten Schenkel (24,26) an der der Fensteröffnung (18; 20) zugewandten Fläche auf einer schraubenförmigen Bahn liegende Nuten (46) aufweist, in die der Gewindesteg (42) der Schraubspindel (38) eingreift.

4. Endoprothese nach mindestens einem der Ansprüche 1 bis 3, dadurch gekennzeichnet, daß die Schraubachse (32) der Schraubspindel (38) bezüglich der Längsachse (34) des Schaftes (12) zum Hals (17) hin unter einem Winkel (a) im Bereich von 2° bis etwa 10° geneigt ist.

5. Endoprothese nach mindestens einem der Ansprüche 1 bis 4, dadurch gekennzeichnet, daß die Achse (32) der Gewindespindel (38) die Längsachse (34) des Schaftes (12) in einem Punkt schneidet, der bezüglich des Schnittpunktes der Längsachse (34) des Schaftes mit der Achse des Halses (17) nach proximal versetzt ist.

6. Endoprothese nach mindestens einem der Ansprüche 2 bis 5, dadurch gekennzeichnet, daß eine die Fensteröffnung (18) oben begrenzende und die beiden zwischen den Schmalseiten (16) und der Fensteröffnung (18) gebildeten Schenkel (24,26) verbindende Brücke (28) eine den Kern (40) der Schraubspindel (38) führende Bohrung (28) aufweist, daß in der Brücke (28) ein in die Bohrung (28) mündender, auf einer schraubenförmigen Bahn liegender Schlitz (36) vorgesehen ist, dessen Steigung und Höhe der Steigung und Dicke des Gewindesteges (42) jeweils entsprechen und der deck- und bodenseitig aus der Brücke (28) austritt, daß die Schraubspindel (38) durch die Brücke (28) hindurch eingeschraubt ist und die mindestens eine Fensteröffnung (18; 20) etwa in Schaftlängsrichtung vollständig durchsetzt.

7. Endoprothese nach Anspruch 6, dadurch gekennzeichnet, daß der Schlitz (36) an beiden Breitseiten (14) des Schaftes (12) offen ist.

8. Endoprothese nach mindestens einem der Ansprüche 1 bis 7, dadurch gekennzeichnet, daß das distale Ende des Kerns (40) der Schraubspindel (38) in einem Sackloch (44) des Schaftes (12) formschlüssig aufgenommen ist.

9. Endoprothese nach mindestens einem der Ansprüche 1 bis 8, dadurch gekennzeichnet, daß der Kern (40) der Schraubspindel (38) durchgehend kreiszylindrisch ist und sein Durchmesser höchstens etwa 30% des größten Durchmessers des Gewindesteges (42) der Schraubspindel (38) beträgt.

10. Endoprothese nach mindestens einem der Ansprüche 1 bis 9, dadurch gekennzeichnet, daß in das proximale Ende des Schaftes (12) im Abstand von der Schraubspindelachse (32) ein Loch (50) vorgesehen ist, das mit einer im Gewindesteg (42) der Schraubspindel (38) vorgesehenen Ausnehmung (46) fluchtet und daß in das Loch (50) ein in die Ausnehmung (46) hineinreichendes Drehsicherungselement (52) eingesetzt ist.

## Claims

1. A cement-free endoprosthesis in particular for hip joints, having a screw spindle (38) which can be screwed into the natural bone channel and which has an outer thread and whose proximal end is mechanically joined to a neck (17) arranged oblique to the spindle axis (32), to form a hinge head, characterised in that the screw spindle (38) is screwed in, approximately in the longitudinal direction of a shaft (12), which shaft is approximately adapted to the bone channel as regards cross-section and which tapers towards the distal end and has two broad sides (14) and two narrow sides (16) joining the latter; in that the outer thread is constructed as a broad-faced thread step (42) projecting from the shaft (12) on both broad sides thereof; and in that it is the shaft (12) which joins the screw spindle (38) to the neck (17).

2. An endoprosthesis according to Claim 1, characterised in that the shaft (12) has at least one window opening (18; 20) which penetrates the shaft and through which the screw spindle (38) extends from proximal to distal; and in that permeable cavities remain between the core (40) of the screw spindle (38) and the arms (24, 26) formed between the narrow sides (16) of the shaft (12) and the window opening (18; 20).

3. An endoprosthesis according to Claim 1 or 2, characterised in that at least one of the arms (24, 26) formed between the narrow sides (16) of the shaft (12) and the window opening (18; 20) has grooves (46) lying on a helical path on the surface facing the window opening (18; 20), into which grooves the thread step (42) of the screw spindle (38) engages.

4. An endoprosthesis according to at least one of Claims 1 to 3, characterised in that the screw axis (32) of the screw spindle (38) is inclined towards the neck (17) relative to the longitudinal axis (34) of the shaft (12) at an angle (a) in the region of 2° to about 10°.

5. An endoprosthesis according to at least one of Claims 1 to 4, characterised in that the axis (32) of the thread step (38) intersects the longitudinal axis (34) of the shaft (12) at a point which is offset towards proximal relative to the point where the longitudinal axis (34) of the shaft intersects the axis of the neck (17).

6. An endoprosthesis according to at least one of Claims 2 to 5, characterised in that a bridge (28), which is the upward limitation of the window opening (18) and which joins the two arms (24, 26) formed between the narrow sides (16) and the window opening (18), has a bore (28) guiding the core (40) of the screw spindle (38); in that a slot (36) is provided in the bridge, which slot opens in the bore (30) and lies on a helical path and whose pitch and height are the same respectively as the pitch and thickness of the thread step (42) and which emerges from the bridge (28) at the top and bottom; in that the screw spindle (38) is screwed in through the bridge (28) and completely penetrates the at least one window opening (18; 20) in approximately the longitudinal direction of the shaft.

7. An endoprosthesis according to Claim 6, characterised in that the slot (36) is open at both broad sides (14) of the shaft (12).

8. An endoprosthesis according to at least one of Claims 1 to 7, characterised in that the distal end of the core (40) of the screw spindle (38) is accommodated in a positive-locking way in a blind hole (44) of the shaft (12).

9. An endoprosthesis according to any one of Claims 1 to 8, characterised in that the core (40) of the screw spindle (38) is continuously circular-cylindrical and its diameter is at most approximately 30% of the greatest diameter of the thread step (42) of the screw spindle (38).

10. An endoprosthesis according to at least one of Claims 1 to 9, characterised in that a hole (50) is provided in the proximal end of the shaft (12) at a distance from the screw spindle axis (32), which hole is in alignment with a recess (46) provided in the thread step (42) of the screw spindle (38); and in that a rotation-prevention member (52) is inserted into the hole (50) and reaches into the recess (46).

**Revendications**

1. Endoprothèse sans ciment, en particulier pour les articulations de la hanche, avec une tige filetée (38), présentant un filet extérieur, à visser dans le canal naturel de l'os, dont l'extrémité proximale est reliée mécaniquement à un col (17) pour une tête d'articulation prévu oblique par rapport à l'axe (32) de la tige, caractérisée en ce que la tige filetée (38) est vissée dans une tige (12), environ dans le sens longitudinal de celle-ci, adaptée environ, en section, au canal de l'os et se rétrécit vers l'extrémité distale, laquelle tige présente deux côtés larges (14) et deux côtés étroits (16) qui les relient, que le filet extérieur est conçu comme spirale (42) à surface large qui sort de la tige (12) à ses deux côtés larges et que la connexion de la tige filetée (38) avec le col (17) se fait par la tige (12).

2. Endoprothèse suivant la revendication 1, caractérisée en ce que la tige (12) présente au moins une ouverture de fenêtre (18, 20) qui la traverse, à travers laquelle s'étend la tige filetée (38) de l'extrémité proximale vers l'extrémité distale et qu'il reste des espaces creux de passage entre le noyau (40) de la tige filetée (38) et les branches (24, 26) formées entre les côtés étroits (16) de la tige (12) et l'ouverture de fenêtre (18, 20).

3. Endoprothèse suivant la revendication 1 ou 2, caractérisée en ce qu'au moins l'une des branches (24, 26) formées entre les côtés étroits (16) de la tige (12) et l'ouverture de fenêtre (18, 20) présente, à la surface tournée vers l'ouverture de fenêtre (18, 20), des rainures (46) suivant un tracé hélicoïdal, dans lesquelles s'engage la spirale (42) de la tige filetée (38).

4. Endoprothèse suivant au moins l'une des revendications 1 à 3, caractérisée en ce que l'axe (32) de la tige filetée (38) est incliné vers le col (17), par rapport à l'axe longitudinal (34) de la tige (12), suivant un angle (a) de l'ordre de 2° à environ 10°.

5. Endoprothèse suivant au moins l'une des revendications 1 à 4, caractérisée en ce que l'axe (32) de la tige filetée (38) vient en intersection avec l'axe longitudinal (34) de la tige (12) en un point décalé vers l'extrémité proximale par rapport au point d'intersection de l'axe longitudinal (34) de la tige avec l'axe du col (17).

6. Endoprothèse suivant au moins l'une des revendications 2 à 5, caractérisée en ce que le pont (28), délimitant du côté supérieur l'ouverture de fenêtre (18) et reliant les deux branches (24, 26) formées entre les deux côtés étroits (16) et l'ouverture de fenêtre (18), présente un orifice (28) guidant le noyau (40) de la tige filetée (38), qu'il est prévu, dans le pont (28) une fente (36) suivant un tracé hélicoïdal aboutissant dans l'orifice (30), dont la montée et la hauteur correspondent chaque fois à la montée et à l'épaisseur de la spirale (42) et qui sort du pont (28) du côté supérieur et du côté inférieur, que la tige filetée (38) est vissée à travers le pont (28) et traverse totalement au moins l'une ouverture de fenêtre (18, 20) environ dans le sens longitudinal de la tige.

7. Endoprothèse suivant la revendication 6, caractérisée en ce que la fente (26) est ouverte des deux côtés larges (14) de la tige (12).

8. Endoprothèse suivant au moins l'une des revendications 1 à 7, caractérisée en ce que l'extrémité distale du noyau (40) de la tige filetée (38) est reçue en liaison de forme dans un trou borgne (44) de la tige (12).

9. Endoprothèse suivant au moins l'une des revendications 1 à 8, caractérisée en ce que le noyau (40) de la tige filetée (38) est totalement cylindrique et que son diamètre est de maximum environ 30% du diamètre le plus grand de la spirale (42) de la tige filetée (38).

10. Endoprothèse suivant au moins l'une des revendications 1 à 9, caractérisée en ce que, dans l'extrémité proximale de la tige (12), à distance de l'axe de la tige filetée (43), il est prévu un orifice (50) qui fuit avec un évidement (46) prévu dans la spirale (42) de la tige filetée (38) et qu'il est placé, dans l'orifice (50), un élément (52) empêchant la rotation qui s'étend jusque dans l'évidement (48).

FIG.1

FIG.2

FIG.3